# EUROPÄISCHE PATENTSCHRIFT

(11) **EP 1 257 293 B1**
(45) Veröffentlichungstag und Bekanntmachung des Hinweises auf die Patenterteilung: **14.09.2005**
(21) Anmeldenummer: 01913654.8
(22) Anmeldetag: 12.02.2001
(51) Int. Cl.: A61K 45/00, A61K 39/40, A61K 39/42, A61P 9/00, A61P 11/00, A61P 17/00, A61P 25/00, A61P 31/00, A61P 37/00

(54) **IMMUNMODULATORISCH WIRKSAME ZUSAMMENSETZUNGEN, VERFAHREN ZU IHRER HERSTELLUNG UND IHRE VERWENDUNG**
IMMUNOMODULATORY EFFECTIVE COMPOSITIONS, METHODS FOR THE PRODUCTION THEREOF AND THEIR USE
COMPOSITIONS A EFFET IMMUNOREGULATEUR, PROCEDES PERMETTANT DE LES PREPARER ET LEUR UTILISATION

(30) Priorität: 14.02.2000 DE 10007771
(43) Veröffentlichungstag der Anmeldung: 20.11.2002
(73) Patentinhaber: Kleine & Steube Entoxin GmbH, 14163 Berlin (DE)
(72) Erfinder: TRENEL, Angela, 30880 Laatzen (DE)
(74) Vertreter: Wehlan, Helmut
(86) Internationale Anmeldenummer: PCT/DE2001/000578
(87) Internationale Veröffentlichungsnummer: WO 2001/058486

(56) Entgegenhaltungen:
- WO-A-98/14212
- DE-A- 3 932 100
- DE-A- 4 236 069
- FR-A- 2 699 822
- US-A- 5 773 570

## Beschreibung

Die Erfindung betrifft immunmodulatorisch wirksame mikrobiologische Zusammensetzungen, Verfahren zu ihrer Herstellung und ihre Verwendung. Die erfindungsgemäßen mikrobiologischen Immunmodulatoren eignen sich zu aktiven und zur passiven Immunisierung. Als homöopathische Arzneimittel liegen ihre Hauptanwendungen auf den Gebieten Herz- und Kreislaufstörungen. Hypertonie oder allergische Leiden.

Immunmodulatorisch wirksame Zusammensetzungen sind in der Patentliteratur mehrfach beschrieben worden. z.B. in der Schrift WO 98/56405 als pharmazeutische Zusammensetzung.

Gegenstand der europäischen Patentanmeldung EP 0 109 089 sind Modulatoren des humanen Immunsystems. die aus Dialysaten von Leukozytextrakten isoliert wurden. In der Schrift WO 94/26114 werden Verfahren und Zusammensetzungen zur Modulierung der Immunantwort erwähnt. Zusammensetzungen zur Behandlung der Immunschwäche, die beispielsweise einen Anticytokin-Antikörper enthalten. sind der internationalen Patentanmeldung WO 94/10980 zu entnehmen. Zusammensetzungen, die Antikörper oder antikörperähnliche Moleküle von primären Antigenen enthalten, beschreibt WO 95/07933. Im Dokument WO 98/52605 wird über eine mögliche immunwirksame Zusammensetzung berichtet, die ein Antigen oder eine antigeninduzierende Substanz sowie einen Träger enthält und eine Steigerung der Immunantwort bewirken soll. Eine Zusammensetzung zur Modulierung der Immunantwort in Säugetieren beschreibt die Patentanmeldung WO 98/57620. Darin wird als therapeutisches Agens ein Stereoisomer von Inositol. dessen Derivate. Salze und Mischungen angegeben. Dieses Agens steigert bzw. unterdrückt die B- und/oder T-Lymphozyten-Aktivität. Gegenstand der Druckschrift WO 98/32431 sind u.a. Antigeninduzierer und Antitoxine, die hohe Selektivität und Spezifität besitzen und als Regulatoren der Immunantwort verwendet werden können. DE 4 236 069 beansprucht ein Arzneimittel bestehend aus einem einen HIV-Erreger und/oder HIV-Antikörper enthaltenden Serum, welches potenziert ist.

Der Erfindung liegt die Aufgabe zugrunde, neue pharmazeutische Zubereitungen zu entwickeln und Verfahren zu ihrer Herstellung anzugeben. Die Aufgabe wurde durch die Bereitstellung immunmodulatorisch wirksamer Zusammensetzungen gelöst. Sie sind dadurch gekennzeichnet. dass sie zu gleichen Teilen aus Antigen- und Antitoxin-Suspensionen bestehen und mit einer Potenzierlösung potenziert werden.

Dabei werden die Antigene aus folgenden Mikroorganismen gewonnen:
- aus Mycobacterium tuberculosis. Mycobacterium tuberculosis typus bovis oder
- Lactococcus lactis
- Virus influencae
- Streptococcus pyogenes, -oralis, -pneumoniae
- Staphylococcus aureus sub. aureus, -epidermidis
- Treponema pallidum
- Plasmodium malaria
- Haemophilus influencae oder
- Klebsiella pneumoniae
und die Antitoxin-Suspensionen durch Immunisierung von nicht-humanen Säugetieren - wie Ratten, Mäuse, Pferde, insbesondere Kaninchen - mit den entsprechenden Antigen-Suspensionen.

Die erfindungsgemäßen Zusammensetzungen enthalten eine Potenzierlösung aus Ethanol und/oder Thymol und gereinigtem Wasser. Zusätzlich können folgende Stoffe in der Potenzierlösung enthalten sein: Jod, Salzsäure und/oder Zuckersirup. Vorzugsweise werden die gleichen Teile der Antigen- und Antitoxin-Suspensionen auf D9 (dil. 9) potenziert.

Die erfindungsgemäßen Zusammensetzungen stellen mikrobiologische Immunmodulatoren dar. Sie enthalten Antigene und Antitoxine verschiedener Bakterienstämme. Die Antitoxin-Suspensionen enthalten eine Phenol-Milchsäure-Mischung.

Das Indikationsspektrum dieser Zusammensetzungen ist außerordentlich breit. Bedingt durch die Tatsache. dass viele Erkrankungen auf Mischinfektionen, Störungen des Immunsystems, Allergien oder auf Autoimmunkrankheiten beruhen, ergibt sich hier eine große therapeutische Breite.

Die Immuntherapie mittels dieser Zusammensetzungen erschließt das Gebiet der Krankheiten mit sogenannten "unbekannten Ursachen". Oft verbergen sich dahinter Tuberkulotoxikosen oder über mehrere Generationen hinweg luetisch-toxische Erbschwächen.

Die erfindungsgemäßen Zusammensetzungen können perkutan zur Anwendung kommen, d. h. sie werden an einer möglichst zarten Hautstelle mit dem Daumenballen vom Patienten selbst kräftig eingerieben. Das können die Ellenbeugen, die Innenseiten der Oberschenkel oder die Bauchhaut sein. Bei Säuglingen können die Tropfen mit dem Unterärmchen des Kindes in die Bauchhaut eingerieben werden.

Das erfindungsgemäße Verfahren zur Herstellung der Zusammensetzungen umfasst folgende Schritte:
a) Herstellung von Antigen-Suspensionen aus abgetöteten Kulturen von Mikroorganismen
b) Herstellung von Antitoxin-Suspensionen durch Immunisierung von nicht-humanen Tieren mit den entsprechenden Antigen-Suspensionen
c) Mischen von Antigen-Suspensionen und den entsprechenden Antitoxin-Suspensionen im Verhältnis 1:1 und Versetzen mit der Potenzierlösung.

Die Potenzierlösung enthält Ethanol und/oder Thymol und gereinigtes Wasser. Sie kann zusätzlich Zuckersirup und/oder ethanolische Jodtinktur und/oder Salzsäure enthalten.

Die Merkmale der Erfindung gehen außer aus den Ansprüchen auch aus der Beschreibung hervor, wobei die einzelnen Merkmale jeweils für sich allein oder zu mehreren in Form von Kombinationen vorteilhafte schutzfähige Ausführungen darstellen, für die mit dieser Schrift Schutz beantragt wird. Die Kombination besteht aus bekannten (Potenzierung, Gewinnung aus Mikroorganismen-Stämmen) und neuen Elementen (Zusammensetzungen, die zu gleichen Teilen aus Antigen- und Antitoxin-Suspensionen bestehen und eine Potenzierlösung enthalten; Phenol-Milchsäure-Mischungen), die sich gegenseitig beeinflussen und in ihrer neuen Gesamtwirkung einen Vorteil (synergistischen Effekt) und den erstrebten Erfolg ergeben, der darin liegt, dass nunmehr mikrobiologische Immunmodulatoren zur aktiven und zur passiven Immunisierung zur Verfügung stehen.

Ein weiteres Kombinationsmerkmal besteht darin, dass die erfindungsgemäßen Lösungen 1 bis 10 untereinander kombiniert gemeinsam zur Anwendung gelangen können.

Die Erfindung soll anhand von Ausführungsbeispielen näher erläutert werden, ohne auf diese Beispiele beschränkt zu sein.

### Ausführungsbeispiele

### Beispiel 1: Herstellung der Antigen-Suspension

Ein o.g. Bakterienstamm wird in einem geeigneten Nährmedium bis auf 10⁷ Keime pro Gramm gezüchtet. Diese Kultur wird nach der Keimzahlbestimmung (Vorschrift V.2.6.12 Eu-AB - Europäisches Arzneibuch) mit 5 ml 1-N-Natriumhydroxid-Lösung (DAB - Deutsches Arzneibuch) 15 min bei 121 °C und 101,325 kPa (1 atm) Druck autoklaviert und anschließend mit IN Salzsäure auf pH 7 eingestellt. Es wird gemäß EuAB (Vorschrift 2.6.1) auf Sterilität geprüft. Diese

Suspension ist die Antigen-Suspension und dient als Urtinktur zur Potenzierung.

### Beispiel 2: Herstellung der Antitoxin-Suspension

A: Zur Herstellung dieser Urtinktur werden drei Kaninchen mit einem Gewicht von jeweils über 2,5 kg für die Immunisierung verwendet. Jedes Kaninchen wird nach zweiwöchiger Quarantäne tierärztlich untersucht und erst nach Ausstellung eines Gesundheitszeugnisses für die Immunisierung freigegeben.

B: Jedem Kaninchen werden 2 ml der "Antigen-Suspension" subkutan verabreicht (Boosterung nach 7 und ggf. weiteren Tagen). Die Immunisierung ist beendet. sobald spezifische Antikörper im Bakterien-Agglutinations-Test nachweisbar sind.

C: Dem Kaninchen mit dem höchsten Antikörpertiter werden lege artis 24 ml Blut entnommen, von denen 22 ml unmittelbar nach der Entnahme in 88.0 ml Phenol-Milchsäure-Mischung (0.5 % verflüssigtes Phenol DAC (Deutscher Arzneimittel Codex). 0,5 % Milchsäure DAB 10 (G/V)). Diese Mischung (110,0 ml) wird zwei Minuten lang leicht geschüttelt.

D: Zu den 110,0 ml der Blut-Phenol-Milchsäure-Mischung nach C werden weitere 990,0 ml der in C beschriebenen Phenol-Milchsäure-Mischung gegeben. Diese Mischung (1100,0 ml - als "Stammlösung" bezeichnet)) wird zwei Minuten lang leicht geschüttelt.

E: 55,0 ml der Stammlösung nach D werden mit 1045,0 ml der in C beschriebenen Phenol-Milchsäure-Mischung verdünnt und 15 min lang bei 121°C und 101,325 kPa (1 atm) Druck autoklaviert. Die Mischung (insgesamt 1100,0 ml) wird gemäß EuAB (Vorschrift V.2.6.1) auf Sterilität geprüft.

F: 1000,0 ml der Mischung nach E werden mit 5,0 ml Salpetersäure 65 % DAB 10 versetzt und während 30 min am Kochen gehalten. Die noch heiße Mischung wird zuerst mit Natriumhydroxid DAB 10 neutralisiert und dann mit Natriumcarbonat DAB 10 auf pH 8.0 eingestellt und nach dem Erkalten mit 10.0 ml Ethanol 96 % DAB 10 und 50.0 ml Glycerol 85 % DAB 10 versetzt. Anschließend wird die Lösung auf 1000,0 ml mit Wasser für Injektionszwecke DAB 10 aufgefüllt. Diese Lösung ist die Antitoxin-Suspension, die als Urtinktur zur Potenzierung dient.

### Beispiel 3: Potenzierung

Die Antigen-Suspension und Antitoxin-Suspension werden zu gleichen Teilen (m/m) gemischt und mit der Potenzierlösung nach den Vorschriften des HAB1 (Homöopathisches Arzneibuch) potenziert.

### Beispiel 4: Lösungen

### Lösung 1

Zusammensetzung: 1ml enthält Antigene dil. D9 und Antitoxine dil. D9 aus Mycobacterium tuberculosis typus bovis.

Anwendungsgebiete: Hypertonie. Herzerkrankungen. vorzeitige Altersbeschwerden bei Drüsen-und Stoffwechselstörungen. Arteriosklerose. Nervenkrankheiten. Parodontose, Prostataerkrankungen.

### Lösung 2

Zusammensetzung: 1ml enthält Antigene dil. D9 und Antitoxine dil. D9 aus Virus influencae, Haemophilus influencae, Klebsiella pneumoniae.

Anwendungsgebiete: Erkältungskrankheiten, Grippe, Angina, Furunkulose. Entzündungen.

### Lösung 3

Zusammensetzung: 1ml enthält Antigene dil. D9 und Antitoxine dil. D9 aus Staphylococcus aureus sub. aureus, Streptococcus pneumoniae.

Anwendungsgebiete: Kreislaufstörungen, venöse Erkrankungen, Koliken, allergische Leiden wie Asthma, Heuschnupfen usw.

### Lösung 4

Zusammensetzung: 1ml enthält Antigene dil. D9 aus Mycobacterium tuberculosis typus bovis, Lactococcus lactis, Streptococcus pyogenes, -oralis, -pneumoniae. Staphylococcus aureus sub. aureus, -epidermis.

Anwendungsgebiete: Kreislaufstörungen, venöse Erkrankungen, allergische Leiden in Kombination mit Lösung 1 und Lösung 3, Schmerzbehandlung.

### Lösung 5

Zusammensetzung: 1ml enthält Antigene dil. D9 und Antitoxine dil. D9 aus Mycobacterium tuberculosis typus bovis, Streptococcus pyogenes.

Anwendungsgebiete: Rheuma. Gicht. Ischias, Neuralgien.

### Lösung 6

Zusammensetzung: 1ml enthält Antigene dil. D9 und Antitoxine dil. D9 aus Mycobacterium tuberculosis, Mycobacterium tuberculosis bovis, Streptococcus pneumoniae.

Anwendungsgebiete: Skrofulose und Tuberkulose sowie deren latente und larvierte Ausdrucksformen wie Asthma, Ekzeme. Rheuma, Migräne usw. Die gemeinsame Anwendung mit Lösung 5 bei rheumatischen Krankheitsbildern ist vorteilhaft. Lösung 6 ist das Mittel der Wahl bei Haut- und Lymphknotenerkrankungen im Kindesalter auf allergischer Grundlage (Skrofulose).

### Lösung 7

### Zusammensetzung:

1ml enthält Antigene dil. D9 und Antitoxine dil. D9 aus Treponema pallidum.

### Lösung 8

### Zusammensetzung:

1ml enthält Antigene dil. D9 und Antitoxine dil. D9 aus Plasmodium malaria.

### Lösung 9

Zusammensetzung: 1ml enthält Antigene dil. D9 aus Mycobacterium tuberculosis typus bovis,

Lactococcus lactis, Streptococcus pyogenes, -oralis, -pneumoniae, Staphylococcus aureus sub. aureus, -epidermidis.

Anwendungsgebiete: Testung aller Herdinfekte an Zähnen. Tonsillen, Nebenhöhlen usw.

### Lösung 10

Zusammensetzung: 1ml enthält Antigene dil. D9 und Antitoxine dil. D9 aus Staphylococcus aureus sub. aureus, Streptococcus pneumoniae.

Anwendungsgebiete: Testung aller Herdinfekte an Zähnen, Tonsillen, Nebenhöhlen usw.

### Literaturhinweis

Die Angaben in den genannten Arzneibüchern
- EuAB: - Europäisches Arzneibuch
- DAB: - Deutsches Arzneibuch
- DAC: - Deutscher Arzneimittel Codex
- HAB: - Homöopathisches Arzneibuch
beziehen sich auf die jeweils gültige Fassung.

## Patentansprüche

1. Immunmodulatorisch wirksame Zusammensetzungen, **dadurch gekennzeichnet, dass** sie zu gleichen Teilen aus Antigen- und Antitoxinsuspensionen bestehen und mit einer Potenzierlösung potenziert werden, wobei die Antigen-Suspensionen aus folgenden Mikroorganismen:
- aus Mycobacterium tuberculosis, Mycobacterium tuberculosis -typus bovis oder
- Lactococcus lactis
- Virus influencae
- Streptococcus pyogenes, -oralis, -pneumoniae
- Staphylococcus aureus sub. aureus, -epidermidis
- Treponema pallidum
- Plasmodium malaria
- Haemophilus influencae oder
- Klebsiella pneumoniae
und die Antitoxin-Suspensionen durch Immunisierung von nicht-humanen Säugetieren, wie Kaninchen, mit den entsprechenden Antigen-Suspensionen gewonnen werden.

2. Zusammensetzungen nach Anspruch 1, **dadurch gekennzeichnet, dass** die Antitoxin-Suspensionen eine Phenol-Milchsäure-Mischung enthalten.

3. Zusammensetzungen nach Anspruch 1 und 2, **dadurch gekennzeichnet, dass** die Phenol-Milchsäure-Mischung 0,5% Phenol und 0,5% Milchsäure enthält.

4. Zusammensetzungen nach Anspruch 1, **dadurch gekennzeichnet, dass** die Potenzierlösung Ethanol und/oder Thymol und gereinigtes Wasser enthält.

5. Zusammensetzungen nach Anspruch 1 und 4, **dadurch gekennzeichnet, dass** die Potenzierlösung zusätzlich Zuckersirup und/oder ethanolische Jodtinktur und/oder Salzsäure enthalten kann.

6. Verfahren zur Herstellung der Zusammensetzungen nach Anspruch 1 bis 5, das folgende Schritte umfasst:
a) Herstellung von Antigen-Suspensionen aus abgetöteten Kulturen von Mikroorganismen gemäß Anspruch 1
b) Herstellung der Antitoxin-Suspensionen durch Immunisierung von nicht-humanen Tieren mit den entsprechenden Antigen-Suspensionen
c) Mischen von Antigen-Suspensionen und den entsprechenden Antitoxin-Suspensionen im Verhältnis 1:1 und Versetzen mit der Potenzierlösung nach Anspruch 4 und 5.

7. Verfahren nach Anspruch 6, **dadurch gekennzeichnet, dass**
a) zur Herstellung der Antigen-Suspensionen Mikroorganismen-Stämme bis auf 10⁷ Keime pro Gramm gezüchtet und diese Kulturen auf pH 7 eingestellt werden
b) zur Herstellung der Antitoxin-Suspensionen die entsprechenden Antigen-Suspensionen den nicht-humanen Tieren subkutan verabreicht werden.

8. Verfahren nach Anspruch 6 und 7, **dadurch gekennzeichnet, dass** zur Herstellung der Antitoxin-Suspensionen Blut, das den nicht-humanen Tieren entnommen wurde, in eine Phenol-Milchsäure-Mischung gemäß Anspruch 2 und 3 aufgenommen wird.

9. Verfahren nach Anspruch 6 bis 8, **dadurch gekennzeichnet, dass** die Suspensionen mittels Milchsäure, Glycerol, Ethanol, Wasser für Injektionszwecke, gereinigtem Wasser, Natriumhydroxid, Natriumcarbonat, Salzsäure, Phenol und Salpetersäure gewonnen werden.

10. Verfahren nach Anspruch 6 bis 9, **dadurch gekennzeichnet, dass** zur Herstellung der Antitoxin-Suspensionen Blut immunisierter nicht-humaner Tiere in einer Phenol-Milchsäure-Mischung autoklaviert, mit Salpetersäure erhitzt, dann neutralisiert, auf pH 8,0 eingestellt, mit Ethanol und Glycerol versetzt und mit Wasser für Injektionszwecke aufgefüllt wird.

11. Verfahren nach Anspruch 6 bis 10, **dadurch gekennzeichnet, dass** die Antigen-Suspension und die Antitoxin-Suspension zu gleichen Teilen gemischt und potenziert werden.

12. Verfahren nach Anspruch 6 bis 11, **dadurch gekennzeichnet, dass** die Potenzierung
a) mittels Ethanol
b) ohne Ethanol und an dessen Stelle mit Thymol, Sirupus simplex, Tinctura Jodi, Acidum hydrochloricum und gereinigtem Wasser vorgenommen wird.

13. Verwendung der Zusammensetzungen nach Anspruch 1 bis 5 zur Herstellung von pharmazeutischen Zubereitungen zur Behandlung von Kreislaufstörungen, Herzerkrankungen, Hypertonie, Entzündungen, Erkältungskrankheiten, Arteriosklerose, allergischen Leiden sowie bei der Testung von Herdinfekten an Zähnen, Tonsillen oder Nebenhöhlen.

14. Verwendung nach Anspruch 13 zur Herstellung von pharmazeutischen Zubereitungen zur Behandlung von venösen Erkrankungen, vorzeitigen Altersbeschwerden bei Drüsen-und Stoffwechselstörungen, Nervenkrankheiten, Parodontose, Prostataerkrankungen, Grippe, Angina, Ekzemen, Furunkulose, Koliken, Asthma, Heuschnupfen, Rheuma, Gicht, Ischias, Neuralgien, Migräne, Skrofulose oder Tuberkulose.

## Claims

1. Immunomodulatory effective compositions, **characterized by** comprising antigen suspensions and antitoxin suspensions in equal parts and are potentiated with a potentiating solution wherein antigen suspensions are obtained from a microorganism of the group consisting of:
- from mycobacterium tuberculosis, mycobacterium tuberculosis typus bovis or
- luctococcus lactis
- virus influenza
- streptococcus pyogenes, -oralis, -pneumoniae
- staphylococcus aureus sub. aureus, -epidermidis
- treponema pallidum
- plusmodium malaria
- haemophilus influenza or
- klebsiella pneumoniae
and the antitoxin suspensions are obtained by immunizing of mammals with the corresponding antigen suspensions.

2. Compositions according to claim 1, wherein the antitoxin suspensions contain a phenol-lactic acid mixture.

3. Compositions according to claim 1 and 2, wherein the phenol-lactic acid mixture contains 0,5 % phenol and 0,5 % lactic acid.

4. Compositions according to claim 1 wherein the potentiating solution contains ethanol and/or thymol and purified water.

5. Compositions according to claim 1 and 4 wherein the potentiating solution contains additionally sugar syrup and/or ethanolic iodine tincture and/or hydrochloric acid.

6. A method for the production of compositions according to claim 1 through 5, comprising the following steps:
a) producing antigen suspensions out of killed cultures of a microorganism according to claim 1
b) producing antitoxin suspensions by immunizing of animals with the corresponding antigen suspensions
c) mixing of antigen suspensions and the corresponding antitoxin suspensions in a ratio 1:1 and furnishing with the potentiating solution according to claim 4 and 5.

7. The method according to claim 6 wherein
a) microorganisms strains are cultivated up to 10⁷ germs per gram for the production of antigen suspensions and these cultures are set to a pH 7
b) the corresponding antigen suspensions are entered subcutaneously to animals for the production of the antitoxin suspensions.

8. The method according to claim 6 and 7 wherein blood, which was removed from non-human animals, is received in a phenol-lactic acid mixture according to claim 2 and 3 for the production of the antitoxin suspensions.

9. The method according to claim 6 through 8 wherein the suspensions are obtained from lactic acid, glycerol, ethanol, water for injection purposes, purified water, sodium hydroxide, sodium carbonate, hydrochloric acid, phenol and nitric acid.

10. The method according to claim 6 through 8 wherein blood of immunized non-human is autoclaved in a phenol-lactic acid mixture, heated with nitric acid, neutralized, set the pH to 8.0; furnished with ethanol and glycerol and filled up with water for injection purposes for the production of the antitoxin suspensions.

11. The method according to claim 6 through 10 wherein the antigen suspension and the antitoxin suspension are mixed in equal parts and are potentiated.

12. The method according to claim 6 through 11 wherein the potentiating step is made
a) with ethanol
b) without ethanol and instead of ethanol with thymol, sirupus simplex, tincture iodine, hydrochloric acid and purified water.

13. Use of compositions according to claim 1 through 5 for manufacturing of pharmaceutical preparations for the treatment of circulatory disorders, cardiac disease, hypertension, inflammations, common colds, arteriosclerosis, allergic ailments as well as during testing of focal infections at teeth, tonsils or nasal sinuses.

14. Use according to claim 13 for manufacturing of pharmaceutical preparations for the treatment of venous diseases, premature geriatric complains in connection with gland disorders and metabolic disorders, neuralgias, paradontosis, prostate disease, influenza, angina, eczema, furunculosis, colics, asthma, hay fever, rheumatism, gout, iscialgia, neuralgia, migraine, scrofulosis or tuberculosis.

## Revendications

1. Compositions agissant de façon immunomodulaire, **caractérisées en ce qu'**elles se constituent pour parties égales de suspensions d'antigène et d'antitoxine et qu'elles peuvent être élevées en puissance par une solution d'élévation en puissance, les suspensions d'antigène étant produites à partir des microorganismes suivants :
- mycobacterium tuberculosis, mycobacterium tuberculosis du type bovis
ou
- lactococcus lactis
- virus influencae
- streptococcus pyogenes, - oralis, - pneumoniae
- staphylococcus aureus sub. aureus, - epidermidis
- treponema pallidum
- plasmodium malaria
- haemophilus influencae ou
- klebsiella pneumoniae
et les suspensions d'antitoxine par immunisation de mammifères non-humains, tels des lapins, avec les suspensions d'antigène correspondantes.

2. Compositions selon la revendication 1, **caractérisées en ce que** les suspensions d'antitoxine contiennent un mélange phénol - acide de lait.

3. Compositions selon les revendications 1 et 2, **caractérisées en ce que** le mélange phénol - acide de lait contient 0,5 % de phénol et 0,5 % d'acide de lait.

4. Compositions selon la revendication 1, **caractérisées en ce que** la solution d'élévation en puissance contient de l'éthanol et/ou du thymol et de l'eau nettoyée.

5. Compositions selon les revendications 1 et 4, **caractérisées en ce que** la solution d'élévation en puissance peut contenir en outre du sirop de sucre et/ou de la teinture d'iode éthanolique et/ou de l'acide chlorhydrique.

6. Procédé de fabrication des compositions selon l'une quelconque des revendications 1 à 5, comportant les étapes suivantes :
a) fabrication de suspensions d'antigène de cultures tuées de microorganismes selon la revendication 1 ;
b) fabrication de suspensions d'antitoxine par immunisation d'animaux non-humains avec les suspensions d'antigène correspondantes ;
c) mélange de suspensions d'antigène et des suspensions d'antitoxine correspondantes dans le rapport 1 : 1 et imprégnation avec la solution d'élévation en puissance selon les revendications 4 et 5.

7. Procédé selon la revendication 6, **caractérisé en ce que**
a) pour la fabrication des suspensions d'antigène, des souches de microorganismes sont élevées jusqu'à obtenir 10⁷ germes par gramme et que le pH de ces cultures est ajusté à 7 ;
b) pour la fabrication des suspensions d'antitoxine, les suspensions d'antigène correspondantes sont fournies de façon subcutanée aux animaux non-humains.

8. Procédé selon les revendications 6 et 7, **caractérisé en ce que**, pour fabriquer des suspensions d'antitoxine, du sang qui fut prélevé sur les animaux non-humains est incorporé dans un mélange phénol - acide de lait selon les revendications 2 et 3.

9. Procédé selon l'une quelconque des revendications 6 à 8, **caractérisé en ce que** les suspensions sont produites au moyen d'acide de lait, de glycérol, d'éthanol, d'eau pour injection, d'eau nettoyée, d'hydroxyde de sodium, de carbonate de sodium, d'acide chlorhydrique, de phénol et d'acide de salpêtre.

10. Procédé selon l'une quelconque des revendications 6 à 9, **caractérisé en ce que**, pour fabriquer des suspensions d'antitoxine, du sang d'animaux immunisés non-humains est autoclavé dans un mélange phénol - acide de lait, chauffé avec de l'acide de salpêtre, ensuite neutralisé, ajusté à un pH de 8,0, imprégné d'éthanol et de glycérol et complété d'eau pour injection.

11. Procédé selon l'une quelconque des revendications 6 à 10, **caractérisé en ce que** la suspension d'antigène et la suspension d'antitoxine sont mélangées à parties égales et élevées en puissance.

12. Procédé selon l'une quelconque des revendications 6 à 11, **caractérisé en ce que** l'élévation en puissance s'effectue :
a) au moyen d'éthanol
b) sans éthanol et, à la place, avec du thymol, sirupus simplex, tinctura iodi, acidum hydrochloricum et de l'eau nettoyée.

13. Utilisation des compositions selon l'une quelconque des revendications 1 à 5 pour la fabrication de préparations pharmaceutiques pour le traitement de dysfonctionnements de la circulation, de maladies cardiaques, d'hypertonies, d'inflammations, de maladies dues à des refroidissements, d'artérioscléroses, de maux allergiques ainsi que lors du test de foyers d'infection des dents, des amygdales ou des sinus paranasales.

14. Utilisation selon la revendication 13 pour la fabrication de préparations pharmaceutiques pour des maladies veineuses, des maladies précoces de vieillesse lors de dysfonctionnements de glandes ou de dysfonctionnements métaboliques, des maladies nerveuses, des parodontoses, des maladies de la prostate, des grippes, des angines, des eczémas, des furonculoses, des coliques, d'asthme, des rhumes des foins, des rhumatismes, des gouttes, des sciatiques, des névralgies, des migraines, des scrofuloses ou des tuberculoses.
